# EUROPEAN PATENT APPLICATION

(11) **EP 1 693 018 A1**
(43) Date of publication of application: **23.08.2006**
(21) Application number: 04806959.5
(22) Date of filing: 13.12.2004
(51) Int. Cl.: A61B 19/00, A61B 17/28

(54) **BENDING OPERATION MEMBER, MULTI-JOINT SLIDER LINK MECHANISM, ACTUATOR, AND MANIPULATOR**

(30) Priority: 11.12.2003 JP 2003413719; 11.12.2003 JP 2003413720; 11.12.2003 JP 2003413721
(71) Applicant: Dohi, Takeyoshi, Tokyo 158-0091 (JP); THK CO., LTD., Shinagawa-ku, Tokyo 141-8503 (JP)
(72) Inventor: DOHI, Takeyoshi, Setagaya-ku, Tokyo 1580091 (JP); HATA, Nobuhiko, Setagaya-ku, Tokyo 1580093 (JP); YAMASHITA, Hiromasa, Kita-ku, Tokyo 1140024 (JP); IIMURA, Akihiro, c/o THK CO., LTD., Shinagawa-ku, Tokyo 1418503 (JP); NAKAZAWA, Toji, c/o THK CO., LTD., Shinagawa-ku, Tokyo 1418503 (JP)
(74) Representative: Grünecker, Kinkeldey, Stockmair & Schwanhäusser Anwaltssozietät
(86) International application number: PCT/JP2004/018598
(87) International publication number: WO 2005/055850

(57) **Abstract**

The front end portion of a bending action member and manipulator is constituted of multi-slider linkage mechanism which starts the operation of a second articulation portion at its rear end side after the bending action of a first articulation portion on the front end side ends. A guide link portion 26 which covers a link member 23 to guide it through a sliding contact is sealed in a hollow portion to prevent leakage of air. In multi-slider linkage mechanism, two frames at a joint portion are formed in forward taper to set the magnitude of a moment required for starting the bending action of a second articulation portion larger than a moment required for the bending action of the first articulation portion. The manipulator is comprised of an actuator portion and a bending forceps portion and capable of engaging/disengaging each other. The actuator portion is provided with a joint arm 13 having a plate containing a fitting hole for connecting the motor with the link member 23. A connecting pin 21 is provided at an end on the engagement/disengagement side of the link member 23 inside the bending forceps portion and fitted to a fitting hole 13c in an elastic body 13a of the joint arm 13, so that the motor and the link member 23 are connectable/separable.

## Description

### TECHNICAL FIELD

The present invention relates to a bending action member, multi-slider linkage mechanism, actuator and manipulator and more particularly the present invention can be preferably applied to a manipulator used in operation such that it is inserted into a space having a higher pressure than the atmospheric pressure. Further, the present invention is preferably applied to a manipulator having multi-slider linkage mechanism which has a plurality of articulation and starts bending action of its rear end side after the bending action of its front end side of the adjoining two articulations ends. Additionally, the present invention is preferably applied to a manipulator used for medical purpose such as minimally invasive surgery.

### BACKGROUND ART

As one of important fields in development of surgery in recent years, the minimally invasive surgery can be mentioned.

That is, in ordinary surgery, normal tissues need to be incised in order to secure an approach path up to arrival at a treatment position and surgery work area as well as the treatment position before executing the medical treatment. For example, when removing the gallbladder, surgical instruments such as scissors need to be inserted into the abdominal cavity by incising the stomach skin and muscle.

Then, a surgery having a decreased damage to a patient or a decreased possibility of giving a damage upon operative treatment is required and the minimally invasive surgery is adopted as an operative treatment capable of suppressing such a damage.

However, the minimally invasive surgery having an advantage of reducing a damage to the patient has various problems for achieving minimally invasive property. Those problems originate from mainly a low degree of freedom possessed by such a surgical instrument such as a laparoscope and long forceps used in operative treatment.

More specifically, because these surgical instruments are introduced into the abdominal cavity through an incised hole in the stomach wall or trocar, an approach to an affected area is limited to a particular direction if considering that points such as the incised hole are substantially fixed. As a consequence, the surgical technique is restricted so that difficulty of surgery is intensified, which is a problem to be solved.

Then, to solve such a problem, various proposals have been made by developing a manipulator by providing a conventional surgical instrument with a new degree of freedom.

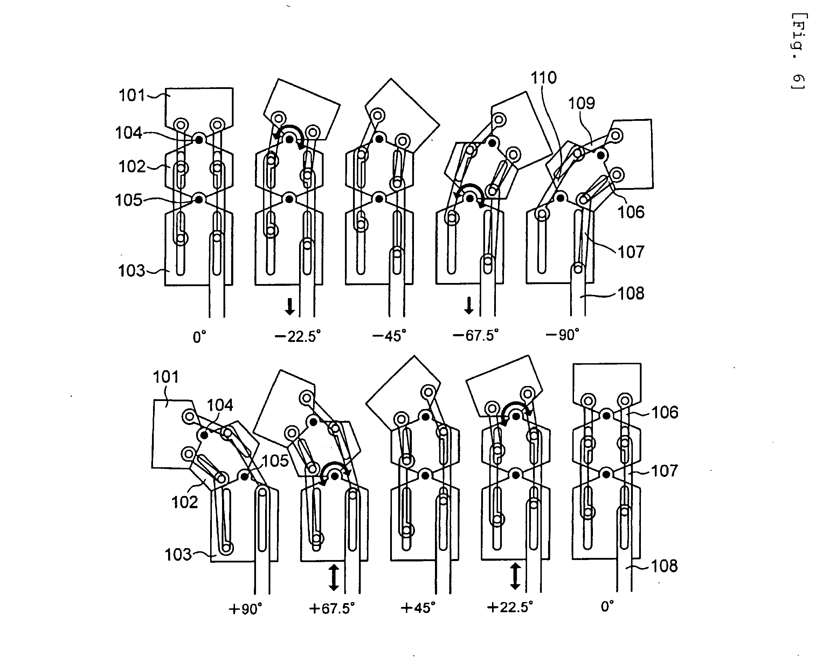

With development of such a manipulator, a forceps manipulator using multi-slider linkage mechanism has been proposed. Portions of the multi-slider linkage mechanism of this forceps manipulator are shown in FIG. 6.

In the forceps manipulator of this related art as shown in FIG. 6, the bending mechanism having a degree of freedom of the slider-link mechanism is constituted of three frames 101, 102, 103, two-pin rotary shafts 104, 105 (2-pin joints) capable of rotating at an angle of ±45°, drive link joints 106, 107, 108, and restraint link joints 109, 110.

A moment about the rotary shaft 104, 105 are given to' the respective frames 101, 102, 103 by sliding the drive link joint 108 in a predetermined direction. The restraint link joints 109, 110 are link joints for restricting the operation so as to allow the second frame to start rotation after the frame 101 at the front end side rotates by ±45°.

Such a structure allows a degree of freedom to bend by ±90° to both sides. Then, a manipulator capable of bending up to 90° independently can be constructed by jointing two bending mechanism with one degree of freedom back and forth so that they are located at 90° each other in the bending direction.

### DISCLOSURE OF THE INVENTION

### PROBLEMS TO BE SOLVED BY THE INVENTION

However, the above-described conventional manipulator has following problems.

When minimally invasive surgery is executed using the manipulator, for example, a manipulator provided with a surgery instrument at a movable portion at its front end is introduced into the abdominal cavity through an incised hole in the stomach wall, the pressure inside the abdominal cavity is set higher than the atmospheric pressure to secure a space for expanding the field of vision and executing operation in order to facilitate the operation.

There was a problem that air in the abdominal cavity leaks when the front end portion of the manipulator is introduced into the abdominal cavity with the inside of the abdominal cavity pressurized. If air in the abdominal cavity leaks, a necessity of pressurizing the abdominal cavity again and always supplying air occurs.

As a result of considering causes for the leakage of air, the inventor of the present invention has found out that a following reason for the leakage of air exists.

To actuate the movable portion constituted of the link mechanism at the front end portion of the above-mentioned manipulator, space (for example, wedge-shaped portion) for bending is required at the bending portion of an adjoining frame and further, a drive power needs to be transmitted to the movable portion at the front end portion to actuate this frame.

To transmit the drive power to this front end portion, as shown in FIG.6, a link member such as a drive link joint 108 is required. Additionally, a pipe having a hollow portion that connects the link member to the movable portion and hold and accommodates this up to its front end portion is necessary.

The inventor of the present invention has paid attention to the restrictions on the structure of the manipulator and found that leakage of air in space having a higher pressure than the atmospheric pressure to outside through the hollow portion in the bent portion of the frame and pipe is one of causes for leakage of air in the abdominal cavity.

The above-mentioned problem occurs not only in the minimally invasive surgery but also in case where the front end portion of the manipulator or the front end portion of a bending action member constructed to be detachable from the manipulator is introduced into space having a higher pressure than the surrounding pressure and driven.

Accordingly, an object of the present invention is to provide a bending action member and manipulator capable of maintaining the pressure of space having a high pressure by preventing air in the high pressure area from leaking to outside through the bending action member or a movable means of the manipulator when the bending action member or the front end portion of the manipulator is introduced into the space having higher pressure than the surrounding pressure.

The conventional multi-slider linkage mechanism and the manipulator having this have following problems.

As described above, the multi-slider linkage mechanism comprises three frames 101, 102, 103 whose end portion is cut out at a predetermined angle and two articulation portions in which these frames 101, 102, 103 are jointed each other.

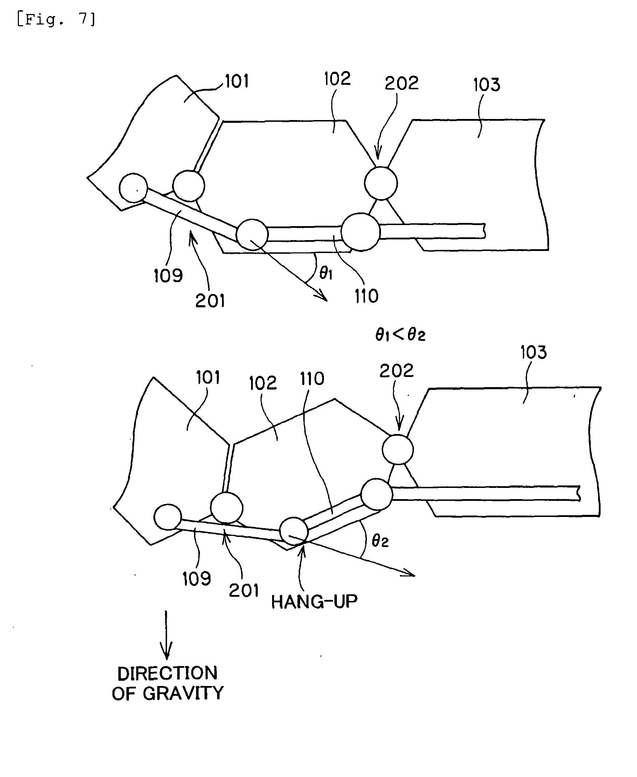

In an ordinary bending action, as shown in FIG. 7A, first, a first articulation portion 201 executes bending action and after the bending action of the first articulation portion 201 ends, the bending action of the second articulation portion 202 is executed. That is, it is so constructed that the bending action of the second articulation portion 202 is not started until the bending action of the first articulation portion 201 is completed.

However, the secondarticulationportion202 is bent before the bending action of the first articulation portion 201 ends by receiving the weights of the frames 101, 102, 103 and other external forces as shown in FIG. 7B, so that it is sometimes bent slightly in an opposite direction with respect to the its proper bending direction of the second articulation portion 202.

When such a bending occurs in the second articulation portion 202, as shown in FIG. 7B, an angle θ₂ between the restraint link 109 and the inner wall surface of the frame 102 becomes larger than an angle θ₁ at the time when the ordinary bending action is carried out as shown in FIG. 7A.

Consequently, a force applied to the inner wall of the frame 102 is increased by the restraint link 109, so that friction force between the restraint link 109 and the frame 102 increases, thereby generating hang-up, namely, phenomenon called "scuffing" in the link portion of the multi-slider linkage mechanism. When this "scuffing" occurs, the multi-slider linkage mechanism itself becomes unmovable, which is a problem to be solved.

Accordingly, a second object of the present invention is to provide a multi-slider linkage mechanism capable of actuating an apparatus having the multi-slider linkage mechanism smoothly and stably by smoothing the operation of the mechanism in order to prevent an unexpected stop of the bending action by suppressing generation of hang-up phenomenon (scuffing phenomenon) in the second articulation portion, which is an articulation on the rear end side of adjoining two articulations, and a bending action member and manipulator.

The conventional manipulator has following problems.

That is, in surgery, the surgical instrument needs to be replaced frequently. Particularly, in minimally invasive surgery, the manipulator used as a surgical instrument needs to be advanced into the human body in minimally invasive condition and operated. Thus, the size thereof increases. Therefore, it is extremely cumbersome to replace such a large manipulator frequently and further, surgery itself is retarded.

The surgical instrument used for such surgery needs to be cleaned and sterilized. However, it is extremely complicated and difficult to clean or sterilize such a large surgical instrument and a manipulator including movable system and drive power system mixedly.

Accordingly, a third obj ect of the present invention is to provide a bending action member which can be replaced effectively in a short time and simplify cleaning work and sterilization work when it is used for surgery or the like, and an actuator and manipulator having the same.

### MEANS FOR SOLVING THE PROBLEM

To achieve the' first object, a first invention of the present invention provides a bending action member comprising:
a movable means in which the front end side portion thereof is bendable;
a drive power transmitting means capable of transmitting a drive power to the movable means;
a member accommodating means that is connected to the movable means and contains a hollow portion accommodating the drive power transmitting means; and
an air-tight member provided in the hollow portion.

According to the first invention, typically, a pair of forceps members constructed that at least one of them is rotatable is provided at the front end of the movable means and the pair of the forceps members is capable of gripping a solid object corresponding to a drive power transmitted by the drive power transmitting means.

A second invention of the present invention provides a manipulator comprising:
a movable means in which the front side portion thereof is bendable;
a drive power generating means capable of generating a drive power for operating the movable means;
a drive power transmitting means capable of transmitting a drive power generated by the drive power generating means to the movable means;
a member accommodating means that is connected to the movable means and contains a hollow portion accommodating the drive power transmitting means; and
an air-tight member provided in the hollow portion.

According to the second invention, typically, a pair of forceps members constructed that at least one of them is rotatable is provided at the front end of the movable means and the pair of the forceps members grips a solid object by transmitting a drive power generated by the drive power generating means with the drive power transmitting means.

According to these first and second inventions, typically, the air-tight member makes a sliding contact with the drive power transmitting means when the drive power is transmitted to the movable means by the drive power transmitting means.

According to these first and second inventions, typically, the movable means is constituted of a plurality of articulation portions and adjoining two articulation portions of the plurality of the articulation portions are so constructed that the articulation portion on the rear end side starts its bending action after the bending of the articulation portion on the front end side ends.

To achieve the second object, a third invention of the present invention provides a multi-slider linkage mechanism further comprising a movable means having a plurality of articulations and constructed to be bendable, wherein
the first articulation portion on the front end side and the second articulation portion on the rear end side that are constructed to be bendable to the same side and adjoin each other of the plurality of the articulation portions are so constructed that the bending action at the second articulation portion starts after the bending action at the first articulation portion ends,
and the magnitude of a moment required for starting the bending action of the second articulation portion is larger than a moment required for the bending action of the first articulation portion.

A fourth invention of the present invention provides a bending action member comprising:
a movable means having a plurality of articulation portions at its front end side portion and constructed to be bendable;
and a drive power transmitting means constructed to be capable of transmitting the drive power to the movable means, wherein
the first articulation portion on the front end side and the second articulation portion on the rear end side that are constructed to be bendable to the same side and adjoin each other of the plurality of the articulation portions are so constructed that the bending action at the second articulation portion starts after the bending action at the first articulation portion ends, and
the magnitude of a moment required for starting the bending action of the second articulation portion is larger than a moment required for the bending action of the first articulation portion.

A fifth invention of the present invention provides a manipulator comprising:
a movable means having a plurality of articulation portions at its front end portion and constructed to be bendable;
a drive power generating means constructed to be capable of generating a drive power for operating the movable means; and
a drive power transmitting means constructed to be capable of transmitting the drive power generated by the drive power generating means to the movable means,
   wherein
the first articulation portion on the front end side and the second articulation portion on the rear end side that are constructed to be bendable to the same side and adjoin each other of the plurality of the articulation portions are so constructed that the bending action at the second articulation portion starts after the bending action at the first articulation portion ends, and
the magnitude of a moment required for starting the bending action of the second articulation portion is larger than a moment required for the bending action of the first articulation portion.

According to these third to fifth inventions, typically, the second articulation portion has a rotary shaft perpendicular to the bending direction of the bending action, and a first frame member and a second frame member having a common rotary shaft and constituting the second articulation portion are so constructed that the first frame member has a first coupling portion; the second frame member has a second joint portion; the first coupling portion and the second coupling portion are jointed with the rotary shaft used in common; and a force generated in the direction along the rotary shaft is increased between the first coupling portion and the second coupling portion accompanied by the bending action of the second articulation portion.

According to these third to fifth inventions, specifically, the contact face of the first coupling portion with the second coupling portion and the contact face of the second coupling portion with the first coupling portion are tapered forward so that the respective contact faces follow each other when it is not bent.

Typically, the third to fifth inventions are so constructed that a moment is applied in the direction that the second articulation portion is restored to a state in which it is not bent, accompanied by the bending action of the second articulation portion.

According to these third to fifth inventions, typically, a pair of forceps members constructed that at least one of them is rotatable is provided at the front end of the movable means and the pair of the forceps members is capable of gripping a solid object by transmitting a drive power generated by the drive power generating means with the drive power transmitting means.

To achieve the third object, the sixth invention of the present invention exists in a bending action member comprising a movable means constructed that its front end side portion is bendable, and a drive power transmitting means constructed to be capable of transmitting a drive power to the movable means, the bending action member being attachable to/detachable from an actuator having a drive power generating means for generating a drive power and the drive power transmitting means being capable of transmitting the drive power from the drive power generating means to the movable means.

According to the sixth invention, typically, the movable means has a plurality of articulations, and the first articulation portion on the front end side and the second articulation portion on the rear end side that are constructed to be bendable to the same side and adj oin each other of the plurality of the articulation portions are so constructed that the bending action at the second articulation portion starts after the bending action at the first articulation portion ends.

According to the sixth invention, typically, the drive power transmitting means is constituted of at least a link member while a first joint portion is constructed of an end on the engagement/disengagement side of the link member and connectable to the drive power generating means through the first joint portion.

This sixth invention is so constructed that the first joint portion has a projecting portion and the projecting portion is capable of being fitted to a fitting hole made in a second joint portion composed of elastic body that has the fitting hole and urges substantially perpendicularly to the transmitting direction of the drive power in the actuator.

The seventh invention of the present invention exists in an actuator comprising a drive power generating means constructed to be capable of generating a drive power for actuating the movable means, the actuator being constructed to be attachable to/detachable from a bending action member having a movable means at its front end side portion.

According to the seventh invention, typically, the drive power generating means has a second joint portion and the second connecting portion which is a drive power generating means is constructed to be connectable to a first joint portion of the drive power transmitting means for transmitting the drive power to the movable means in the bending action member.

According to the seventh invention, typically, the second joint portion in the actuator includes an elastic body containing a fitting hole and for urging substantially perpendicularly to the transmitting direction of the drive power and the second joint portion in the actuator is advanced substantially linearly by the drive power generating means toward the first joint portion of the bending action member having a projecting portion capable of being fitted to the fitting hole and an elastic body generates an urging force to the projecting portion so that the fitting hole is fitted to the projecting portion.

The eighth invention of the present invention exists in a manipulator comprising: a bending action member including a movable means constructed that its end side portion is bendable and a drive power transmitting means capable of transmitting a drive power applied from outside to the movable means; and
an actuator having a drive power generating means capable of generating a drive power for operating the movable means, wherein
the bending action member and the actuator are constructed to be capable of engaging/disengaging each other; the second joint portion of a drive power generating means and the first joint portion of a drive power transmitting means are constructed to be connectable.

According to the eighth invention, typically, the drive power generating means and the drive power transmitting means are constructed to be jointed after the bending action member and actuator are connected to each other and the joint between the drive power generating means and the drive power transmitting means is separable accompanied by the separation between the bending action member and the actuator.

According to the eighth invention, typically, the second joint portion in the actuator includes an elastic body containing a fitting hole and for urging substantially perpendicularly to the transmitting direction of the drive power and the first joint portion in the bending action member has a projecting portion capable of being fitted to the fitting hole and the second joint portion in the actuator being advanced substantially linearly by the drive power generating means, so that the drive power transmitting means and the drive power generating means are connected while an urging force is generated and fitted to the projecting portion.

The technical philosophy of the present invention is not always restricted to the above-described combinations but includes technical philosophies achieved by combining the above-mentioned plural inventions appropriately and arbitrarily.

### EFFECTS OF THE INVENTION

As described above, the bending action member of the first invention and the manipulator of the second invention can reduce or prevent leakage of air in a sealed area to outside when the movable portion of the manipulator or the bending action member is inserted into a substantially sealed space having a higher pressure than the atmospheric pressure and operated. As a consequence, complication of pressurizing air in the sealed area again or continuing to supply air into the inside of the sealed area can be reduced.

The manipulator of the second invention can prevent leakage of air in the abdominal cavity of a patient to outside upon minimally invasive surgery in which medical operation is executed with the abdominal cavity of the patient kept at a higher pressure than the atmospheric pressure. Thus, the minimally invasive surgery by an operator can be facilitated with the safety.

As described above, the multi-slider linkage mechanism of the third invention, the bending action member of the fourth invention and the manipulator of the fifth invention prevent generation of hang-up phenomenon (scuffing phenomenon) at the second articulation portion on the rear end side of adjoining two articulations constructed to bend in the same direction thereby blocking an unexpected stop of the bending action and smoothing the operation of the mechanism. As a result, an apparatus including the multi-slider linkage mechanism can be operated smoothly and stably.

The multi-slider linkage mechanism of the third invention, the bending action member of the fourth invention and the manipulator of the fifth invention prevent hang-up (scuffing) phenomenon in the multi-slider linkage mechanism of the bending action member or the manipulator used in the minimally invasive surgery thereby actuating the mechanism smoothly and stabilizing the operation of the bending action member and the manipulator. As a consequence, the minimally invasive surgery by an operator can be facilitated.

As described above, the bending action member of the sixth invention and the actuator of the seventh invention allow replacement of the bending action member to be carried out effectively in a short time and further, cleaning work and sterilization work to be simplified if the bending action member is used for surgery or the like. Further, the minimally invasive surgery by an operator can be facilitated with the safety.

### BEST MODE FOR CARRYING OUT THE INVENTION

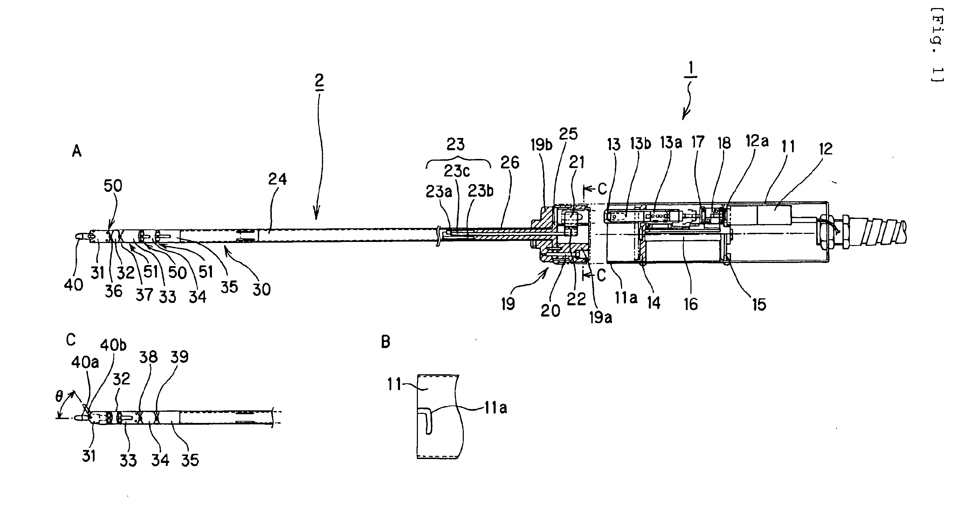

Hereinafter, a manipulator according to an embodiment of the present invention will be described with reference to the accompanying drawings. FIG. 1 shows the entire structure of a manipulator of this embodiment.

### (Manipulator)

As shown in FIG. 1A, the manipulator of this embodiment comprises an actuator portion 1 as a drive power generating means and a joint type bending forceps portion 2.

The actuator portion 1 is constituted of mainly stainless steel (SUS304) and comprises an outer case 11 as a casing, a motor with three reducers provided on three motor bases 12a, three joint arms 13 provided with a connecting plate 13a and joint spring 13b, an arm guide 14 for guiding the joint arms 13, a bearing base 15, a guide base 16, bearing case 17 and a coupling 18.

As shown in FIGS. 1A and 1B, a joint guide groove 11a for jointing with a joint portion 19 in the joint type bending forceps portion 2 is formed for example, in an L shape on the side of the joint portion 19 of the outer case 11 of the actuator portion 1.

The actuator portion 1 is constructed to generate a drive power with the motor 12 with the reducers. Then, this drive power is transmitted to the joint arm 13 through the bearing case 17 and the coupling 18.

This joint arm 13 transmits a drive power to the forceps which is a movable portion at the front end of the joint type bending forceps portion 2. The joint arm 13 is constructed to be movable in the longitudinal direction of the actuator portion 1 corresponding to drive accompanied by generation of a drive power.

The joint type bending forceps portion 2 is constituted by connecting a bending forceps portion 30 having a gripping portion as a forceps member comprised of the multi-slider linkage mechanism at the front end side and the joint portion 19 for connecting to the actuator portion 1 at the other end side with a frame 24 having a hollow portion.

The joint portion 19 includes a joint case 19a, a grip base 19b and an engagement/disengagement pin 20 fitted to the joint guide groove 11a as a fastening device used for engaging with/disengaging from the actuator 1.

Three pairs of connecting pin 21 and locate base 22, which are constructed to be connectable with the above-described three joint arms 13, are provided inside the joint case 19a. These three pairs of the connecting pin 21 and the locate base 22 are provided at an end of the joint portion 19 side for three link members 23 for transmitting each drive power to the bending forceps portion 30 at the front end side.

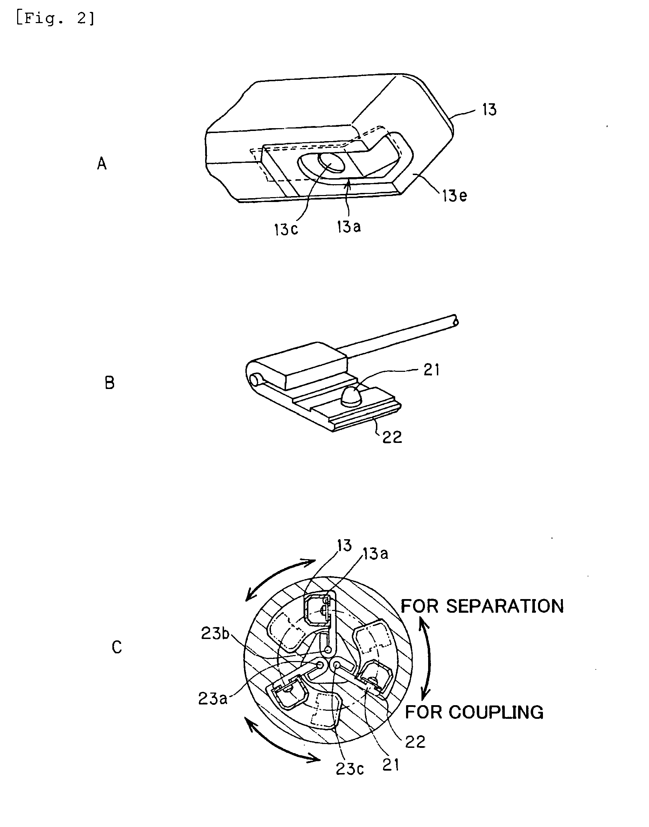

Here, coupling of the connecting pin 21, the locate base 22 and the joint arm 13 will be described with reference to Figures. FIG. 2A shows the joint arm 13 of this embodiment, FIG. 2B shows the connecting pin 21 and locate base 22 at an end of the link member 23 of this embodiment, and FIG. 2C shows a sectional view taken along the line C-C of FIG.1 about the positional relation among the connecting pin 21, the locate base 22 and the joint arm 13 upon separation and coupling.

As shown in FIG. 2A, the joint arm 13 of this embodiment is comprised of the connecting plate 13a and an arm main body 13e. The connecting plate 13a is a plate-like member in which the portion outside the arm main body 13e is bent into a V shape providing a mountain-like shape, having an opening 13c made in the vicinity of an end on the side of the joint case 19a. Further, the connecting plate 13a is constructed to sag with an urging force in a direction substantially vertical to a plate-like face.

As shown in FIG. 2B, the locate base 22 of this embodiment is constructed in the form of a plate and the portion provided with the connecting pin 21 is formed in a convex shape having a step. An end portion of this plate-like locate base 22 is bent to fix an end portion of the link member 23 and connected to an end portion of the link member 23. According to this embodiment, the connecting pin 21 is provided substantially in the central portion of a face formed with a step of the locate base 22. That is, this connecting pin 21 is provided at a position which allows it to be fitted to the opening 13c in the connecting plate 13a of the joint arm 13.

### (Connection between the actuator portion and the joint type bending forceps portion)

Next, fitting between the connecting pin 21 and the opening 13c will be described.

First, the actuator portion 1 is connected to the joint type bending forceps portion 2. That is, the engagement/disengagement pin 20 of the joint type bending forceps portion 2 is inserted into the joint guide groove 11a (refer to FIG. 1B) of the actuator portion 1. As a result, the joint arm 13 advances into the interior of the joint portion 19 of the joint type bending forceps 2. The positional relation among the connecting pin 21, the locate base 22 and the joint arm 13 at this time is indicated with dotted line (at the time of separation) in FIG. 2C.

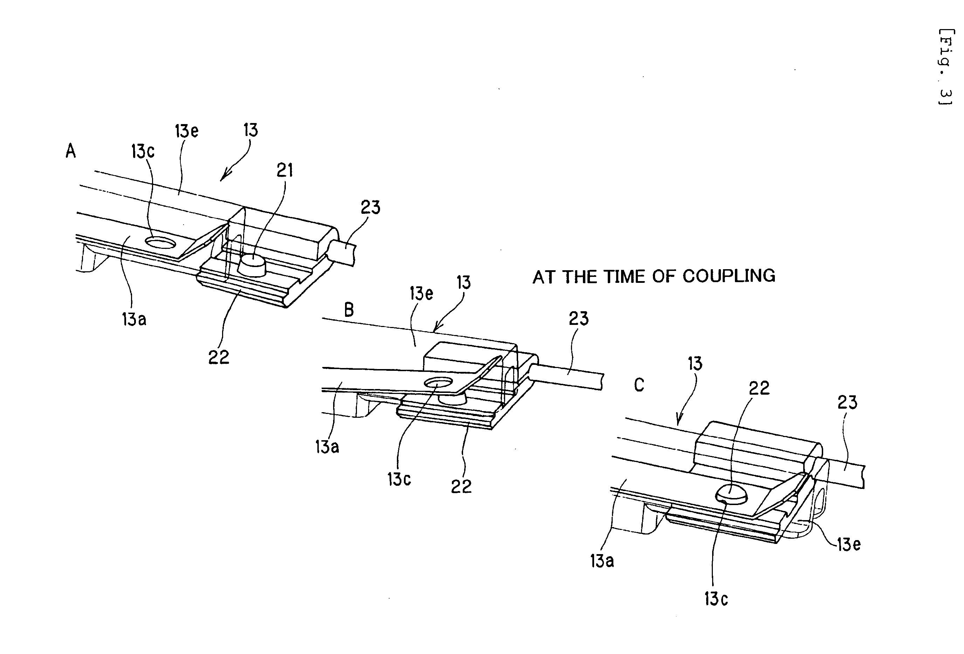

The actuator portion 1 and the joint type bending forceps portion 2 rotate relatively about the central axis when it is advanced further along the shape of the joint guide groove 11a. As a consequence, the positional relation among the connecting pin 21, the locate base 22 and the joint arm 13 as seen from the section is indicated with solid line (coupling side) in FIG.2C. Only when the actuator portion 1 and the joint type bending forceps portion 2 are rotated relative to each other about their central axis and jointed, the connecting pin 21 is not fitted to the opening 13c. The state at this time is indicated in FIG. 3A.

In an initial state in which the actuator portion 1 and the joint type bending forceps 2 are jointed as shown in FIG. 3A, the connecting pin 21 and the opening 13c are located on the same plane and however, they are not fitted to each other.

After that, the joint arm 13 is advanced to the side of the connecting pin 21 by driving the motor 12 with reducer as shown in FIG. 3B. With an advance of this joint arm 13, the portion on the side of the connecting plate 13a of the arm main body 13e is guided by the step portion of the locate base 22 and advanced. At the same time, the front end portion of the connecting plate 13a is brought up such that the top end of the connecting pin 21 makes sliding contact thereby being sagged generating an elastic force.

When the joint arm 13 is advanced further after that, the connecting pin 21 meets the opening 13c as shown in FIG. 3C, so that the connecting pin 21 is fitted to the opening 13c in the connecting plate 13a by an elastic force generated in the connecting plate 13a.

The connecting pin 21 and the opening 13c are fitted to each other by such a series of actions so that the joint arm 13 and the locate base 22 are jointed together. As a consequence, a drive power generated by the motor 12 with reducer can be transmitted to the link member 23 through the joint arm 13 and the locate base 22.

To separate the actuator portion 1 from the joint type bending forceps portion 2, an opposite action to the above-described coupling is carried out. That is, the actuator portion 1 and the joint type bending forceps 2 are rotated about their central axis in the direction for separation shown in FIG. 2C. As a result, the joint arm 13 is raised to the side of the projection of the connecting pin 21 on the locate base 22 and then, the fitting between the connecting pin 21 and the opening 13c is released. After that, the engagement/disengagement pin 20 is moved in the direction of disengagement along the shape of the joint guide groove 11a. Consequently, the actuator portion 1 and the joint type bending forceps 2 are separated.

Ordinarily, the surgical instrument needs to be replaced frequently upon operative treatment. By constructing the actuator portion 1 and the joint type bending forceps portion 2 so as to be connectable and replaceable as described above, only the joint type bending forceps 2 can be replaced as an end effector portion without replacing the actuator portion 1 having a weight. Thus, a necessary tool can be replaced effectively in a short time.

Further, the actuator portion 1 can be used in common for plural kinds of the end effector portions by constructing the joint portion 19 at the end effector portion such as the joint type bending forceps 2 in the above-described way, thereby achieving reduction of cost. Additionally, cleaning and sterilization can be executed easily by constructing the end effector portion and the actuator portion 1 separably.

Under a condition in which the actuator portion 1 and the joint type bending forceps portion 2 are jointed together, the three link members 23 for transmitting a drive power of the motor 12 with reducer are accommodated in the cylindrical frame 24 having a hollow portion.

### (Gas leakage preventing mechanism)

A link base 25 composed of for example, polycarbonate (PC) for holding the link member 23 is provided inside a coupling portion between this frame 24 and the joint portion 19.

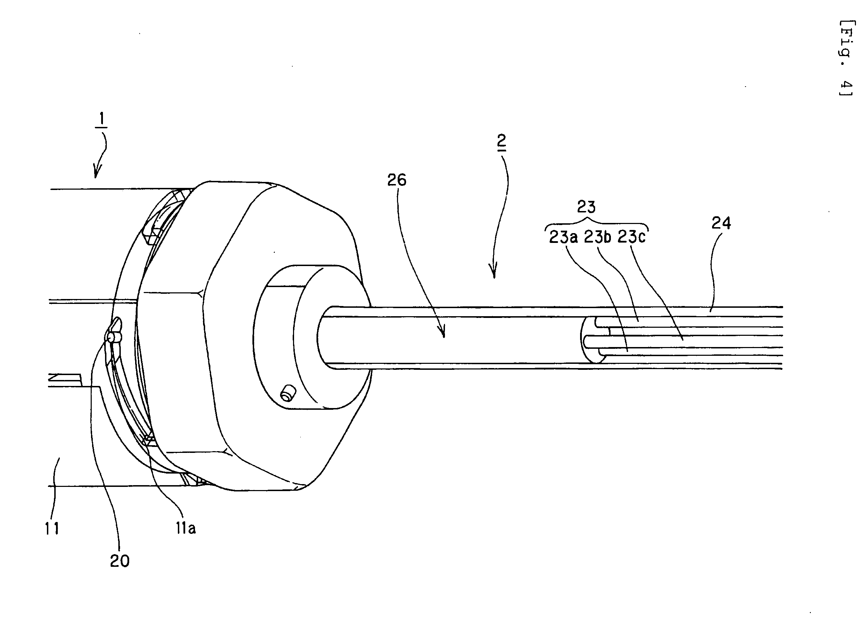

As shown in FIG. 4, a link guide portion 26 is sealed from the grip base 19b up to halfway in the direction to the front end side inside the frame 24. The link guide portion 26 is intended to hold the link members 23a, 23b, 23c and to block fluid as gas invading from the bending forceps 30 at the front end side. As a consequence, leakage of gas to outside through the frame 24 and the joint portion 19 can be blocked inside the frame 24.

When the link members 23a, 23b, 23c transmit a drive power along the longitudinal direction of the frame 24, these link members 23a, 23b, 23c make slide contact with the link guide portion 26. Thus, as material of this link guide portion 26, material having a high durability against sliding contact with the link member 23 and a low gas permeability (gas permeability), for example, polycarbonate (PC), is adopted.

### (Multi-slider linkage mechanism)

The bending forceps 30 on the movable side to which the drive power is transmitted by the link members 23 accommodated in the frame 24 having an improved air-tightness is constructed in multi-slider linkage mechanism. That is, the bending forceps 30 at the front end side is constructed to be bendable with permeation of gas inside the frame 24 is blocked by transmission of the drive power by the link member 23 so as to maintain air-tightness.

More specifically, a first frame 31, a second frame 32, a third frame 33, a fourth frame 34 and a fifth frame 35 are connected in line along the same axis in the bending forceps 30 with the manipulator according to this embodiment.

The first frame 31 and the second frame 32 are connected with the first frame pin 36 so as to constitute a first articulation portion 50. The second frame 32 and the third frame 33 are connected with the second frame pin 37 so as to constitute a second articulation portion 51. Then, the first articulation portion 50 and the second articulation portion 51 are constructed to be bendable along the same plane.

More specifically, the first articulation portion 50 is constructed to be bendable up to, for example, 45° and have the first flame pin 36 as a rotary shaft. The second articulation portion 51 is constructed to be bendable up to, for example, 45° in the same direction as the bending of the first articulation portion.

Thus, the third frame 33 to the first frame 31 are constructed so as to be bendable up to, for example, 90° with the first articulation portion 50 and the second articulation portion 51. Further, because the first articulation portion 50 and the second articulation portion 51 constitute the multi-slider linkage mechanism, the bending action of the second articulation portion 51 is blocked from starting until the bending action of the first articulation portion 50 is ended.

The third frame 33 and the fourth frame 34 at the rear end side are connected with the third frame pin 38. The fourth frame 34 and the fifth frame 35 are connected with the guide pin 39. Then, the third frame 33, the fourth frame 34 and the fifth frame 35 are constructed to be bendable in a direction along the same plane at each coupling portion with the third frame pin 38 and the guide pin 39 used as a rotary shaft.

That is, the third frame 33 to the fifth frame 35 are also constituted of multi-slider linkage mechanism having the first articulation portion 50 and the second articulation portion 51 like the first frame 31 to the third frame 33.

The bending direction of the multi-slider linkage mechanism comprised of the third frame 33 to the fifth frame 35 and the bending direction of the multi-slider linkage mechanism comprised of the first frame 31 to the third frame 33 are constructed to be perpendicular to each other.

The degree of freedom in bending action is intensified by combining bending along a predetermined face at the front end of the bending forceps portion 30 with bending along a plane perpendicular to that plane.

### (Joint structure of second articulation portion)

In the multi-slider linkage mechanism having the first articulation portion 50 and the second articulation portion 51, as described above, the bending action of the second articulation portion 51 is not started until the bending action of the first articulation portion 50 is completed and the bending direction of the first articulation portion 50 is equal to the bending direction of the second articulation portion 51. However, because the bending forceps 30 receives its own weight and other external force from outside, sometimes the second articulation portion 51 is bent before the bending action of the first articulation portion 50 is ended and the second articulation portion 51 is bent slightly in an opposite direction to its original bending direction corresponding to the bending action of the first articulation portion 50.

If such a phenomenon occurs in this second articulation portion 51, hang-up or phenomenon called "scuffing" sometimes occurs in the link portion of the multi-slider linkage mechanism, so that the bending forceps portion 30 itself becomes unmovable depending on a case.

Although ordinarily, use of lubricant can be considered to prevent such "scuffing", lubricant cannot be used at a movable portion of the first articulation portion 50 or the second articulation portion 51 if the manipulator of this embodiment is used for minimally invasive surgery and thus, non-lubrication action needs to be executed.

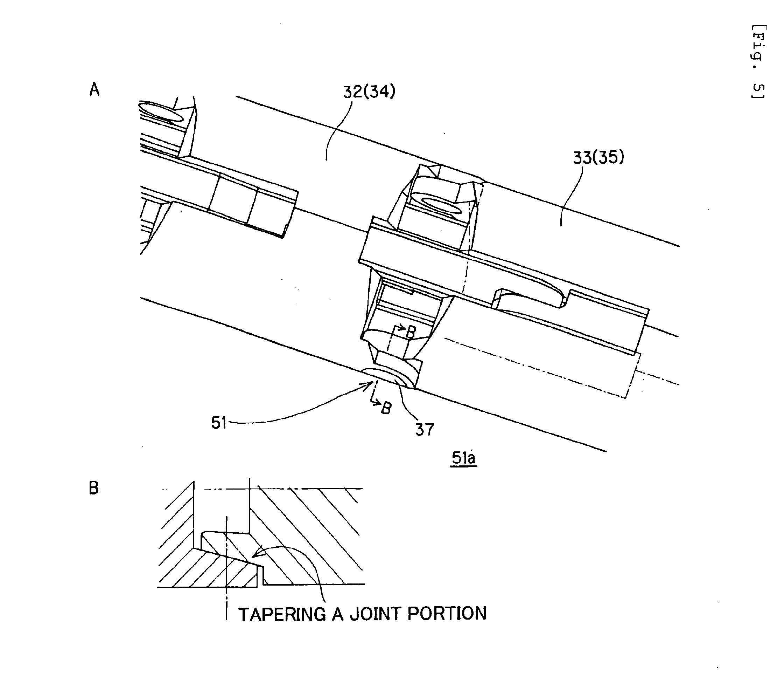

Then, the structure of preventing the scuffing phenomenonofthemulti-sliderlinkagemechanism without use of lubricant in the second articulation portion 51 of this embodiment will be described. FIG. 5A shows the second articulation portion 51 and FIG. 5B shows a partial sectional view taken along the line B-B of the joint portion 51a of the second articulation portion 51. Although in a following description, the second articulation portion 51 comprised of the second frame 32 and the third frame 33 will be described by way of example, the same structure is adopted in the second articulation portion 51 comprised of the fourth frame 34 and the fifth frame 35.

In the second articulation portion 51 of this embodiment, as shown in FIG. 5A, a connecting end of each of the second frame 32 and the third frame 33 is formed into a ring shape and they are jointed with the second frame pin 37 so as to constitute the joint portion 51a.

As shown in FIG. 5B, the ring-like portion in which the second frame 32 and the third frame 33 are jointed with the second frame pin 37 is constructed in a tapered configuration at this joint portion 51a.

More specifically, the inner contact face of the joint portion 51a with the third frame 33 in the second frame 32 is formed in forward taper whose thickness increases as it reaches its root and the outer contact face in contact with the joint portion 51a of the third frame 33 is formed in the forward taper so as to follow up the forward taper at the joint portion 51a of the second frame 32 when they are not bent.

If the second articulation portion 51 is bent with the second frame pin 37 as a shaft at the joint portion 51a in which the two frames are formed in the forward taper, thicker portions in the forward tapered shape of the second frame 32 and the third frame 33 overlap each other accompanied by the advancement of the bending action.

If the thicker portions in the forward tapered shape come to overlap each other accompanied by the bending action, its force increases as compared with a force acting between the two members at the joint portion 51a when the articulation portion is not bent (bending angle: 0°) so that a dynamically unstable state is produced.

That is, with progress of the bending action of the second articulation portion 51, a force acting on the inside of the joint portion 51a of the second frame 32 increases and at the same time, a force acting on the outside of the joint portion 51a of the third frame 33 increases. As a consequence, the force at the joint portion 51a increases so that dynamic energy increases thereby producing a dynamically unstable state.

Ordinarily, the dynamic energy acts so that its magnitude minimizes. Thus, a moment is applied to the joint portion 51a in the direction of minimizing the interacting force when the second articulation portion 51 is bent and accordingly, the magnitude of a moment required for the bending action of the second articulation portion 51 increases as compared with the magnitude of a moment for bending the first articulation portion 50.

As a consequence, a force is generated at the second articulation portion 51 in the direction of restoration to a state in which it is not bent. Thus, a undesired bending action becomes unlikely to occur at the second articulation portion 51, so that the bending action cannot be started unless a larger force than a predetermined force is applied from outside.

Thus, generation of the undesired bending action can be suppressed at the second articulation portion 51 by forming the joint faces (contact faces) at the joint portion 51a of the second frame 32 and the third frame 33 in forward taper to follow up the shape of each other when the articulation is not bent. As a consequence, the hang-up of the second articulation portion 51 can be prevented thereby smoothing and stabilizing the action of the bending forceps portion 30.

In addition to the above-described method of forming the joint face (contact face) of the joint portion 51a of the second articulation portion 50 in forward taper, it is permissible to adopt a variety of methods of applying a force for maintaining the second articulation portion 51 in a state whose bending angle is 0° by setting the magnitude of a force required for bending the second articulation portion 51 larger than a force required for bending the first articulation portion 50.

More specifically, it is permissible to adopt a method of intensifying a force required for the bending by constructing the joint portion 51a at the second articulation portion 51 by interference fit between the frames or a method of constructing the joint portion 51a of the second articulation portion 51 using a spring washer.

Further, to suppress the cause for the scuffing phenomenon, it is permissible to adopt a method of minimizing a friction force at a contact portion between the second frame 32 constituting the second articulation portion 51 and the movable link or the restraint link (both of which is not shown) for bending the second articulation portion 51. More specifically, it is permissible to use a material having a higher hardness than the material constituting the frame, as the material which constitutes the movable link or the restraint link or carry out surface treatment on the link member.

The front end of the bending forceps portion 30 has a movable forceps 40 which is opened at an angle of, for example, 60° and capable of gripping a solid object of a predetermined size which can be opened or closed. This movable forceps 40 comprises a fixed gripping tooth 40a and a movable gripping tooth 40b.

The fixed gripping tooth 40a is fixed to the front end side of the first frame 31. On the other hand, the movable gripping tooth 40b is capable of being opened up to a predetermined angle θ (for example, about 60°) and closed with an elastic body such as a spring (not shown) and by operation through one link member (for example, link member 23c) of the above-described three link members 23.

The manipulator of this embodiment is constituted in the above-described way.

In the meantime, the present invention can be applied to not only the manipulator but also any apparatus which operates by inserting its front end portion into a high pressure portion and transmitting a drive power through link members, the front end portion being provided with a movable portion, in an area having a higher internal pressure than the atmospheric pressure.

The present invention can be applied to not only the manipulator but also any apparatuses having the multi-slider linkage mechanism.

### BRIEF DESCRIPTION OF THE DRAWINGS

[FIG. 1] FIG. 1 is a sectional view showing a manipulator according to an embodiment of the present invention, a side view showing an outer case and a sectional view showing its front end portion.
[FIG. 2] FIG. 2 is a perspective view showing a joint arm in the manipulator according to the embodiment of the present invention, a perspective view showing a connecting pin for connecting, and a sectional view showing a positional relation between the actuator portion and the joint portion at the time of joint and separation.
[FIG. 3] FIG. 3 is a perspective view showing a method for joining the actuator portion and the joint portion in the manipulator according to the embodiment of the present invention.
[FIG. 4] FIG. 4 is a perspective sectional view showing a link guide portion in the frame of the manipulator according to the embodiment of the present invention. [FIG. 5] FIG. 5 is a perspective view showing a second articulation portion equipped in the manipulator according to the embodiment of the present invention and a sectional view showing a joint portion.
[FIG. 6] FIG. 6 is a diagram for explaining the multi-slider linkage mechanism of a related art.
[FIG. 7] FIG. 7 is a diagram for explaining hang-up phenomenon (scuffing phenomenon) in the multi-slider linkage mechanism of the related art.

### DESCRIPTION OF REFERENCE NUMERALS

1: Actuator portion
2: Joint type bending forceps portion
11: Outer case
11a: Joint guide groove
12: Motor with reducer
12a: Motor base
13: Joint arm
13a: Connecting plate
13b: Joint spring
13c: Opening
13e: Arm main body
14: Arm guide
15: Bearing base
16: Guide base
17: Bearing case
18: Coupling
19: Joint portion
19a: Joint case
19b: Grip base
20: Engagement/disengagement pin
21: Connecting pin
22: Locate base
23, 23a, 23b, 23c: Link member
24: Frame
25: Link base
26: Link guide portion
30: Bending forceps portion
31: First frame
32: Second frame
33: Third frame
34: Fourth frame
35: Fifth frame
36: Fist frame pin
37: Second frame pin
38: Third frame pin
39: Guide pin
40: Movable forceps
40a: Fixed gripping tooth
40b: Movable gripping tooth
50: First articulation portion
51: Second articulation portion
51a: Joint portion
101, 102, 103: Frame
104, 105: Rotary shaft
106, 107, 108: Drive link joint
108: Drive link joint
109, 110: Restraint link joint

## Claims

1. A bending action member comprising:
a movable means in which the front end side portion thereof is bendable;
a drive power transmitting means capable of transmitting a drive power to the movable means;
a member accommodating means that is connected to the movable means and contains a hollow portion accommodating the drive power transmitting means; and
an air-tight member provided in the hollow portion.

2. The bending action member according to claim 1 wherein the air-tight member makes a sliding contact with the drive power transmitting means when the drive power is transmitted to the movable means by the drive power transmitting means.

3. The bending action member according to claim 1 or 2 wherein the movable means is constituted of a plurality of articulation portions and adjoining two articulation portions of the plurality of the articulation portions are so constructed that the articulation portion on the rear end side starts its bending action after the bending of the articulation portion on the front end side ends.

4. The bending action member according to any one of claims 1 to 3 wherein a pair of forceps members constructed that at least one of them is rotatable is provided at the front end of the movable means and
the pair of the forceps members is capable of gripping a solid object corresponding to a drive power transmitted by the drive power transmitting means.

5. A manipulator comprising:
a movable means in which the front side portion thereof is bendable;
a drive power generating means capable of generating a drive power for operating the movable means;
a drive power transmitting means capable of transmitting a drive power generated by the drive power generating means to the movable means;
a member accommodating means that is connected to the movable means and contains a hollow portion accommodating the drive power transmitting means; and
an air-tight member provided in the hollow portion.

6. The manipulator according to claim 5 wherein the air-tight member makes a sliding contact with the drive power transmitting means when the drive power is transmitted to the movable means by the drive power transmitting means.

7. The manipulator according to claim 5 or 6 wherein the movable means is constituted of a plurality of articulation portions and adjoining two articulation portions of the plurality of the articulation portions are so constructed that the articulation portion on the rear end side does not start its bending action until the bending of the articulation portion on the front end side ends.

8. The manipulator according to any one of claims 5 to 7 wherein a pair of forceps members constructed that at least one of them is rotatable is provided at the front end of the movable means and
the pair of the forceps members grips a solid object by transmitting a drive power generated by the drive power generating means with the drive power transmitting means.

9. A multi-slider linkage mechanism further comprising a movable means having a plurality of articulations and constructed to be bendable, wherein
the first articulation portion on the front end side and the second articulation portion on the rear end side that are constructed to be bendable to the same side and adjoin each other of the plurality of the articulation portions are so constructed that the bending action at the second articulation portion starts after the bending action at the first articulation portion ends,
and the magnitude of a moment required for starting the bending action of the second articulation portion is larger than a moment required for the bending action of the first articulation portion.

10. The multi-slider linkage mechanism according to claim 9, wherein
the second articulation portion has a rotary shaft perpendicular to the bending direction of the bending action,
and a first frame member and a second frame member having a common rotary shaft and constituting the second articulation portion are so constructed that
the first frame member has a first coupling portion;
the second frame member has a second joint portion;
the first coupling portion and the second coupling portion are jointed with the rotary shaft used in common;
and a force generated in the direction along the rotary shaft is increased between the first coupling portion and the second coupling portion accompanied by the bending action of the second articulation portion.

11. The multi-slider linkage mechanism according to claim 10 wherein the contact face of the first coupling portion with the second coupling portion and the contact face of the second coupling portion with the first coupling portion are tapered forward so that the respective contact faces follow each other when it is not bent.

12. A bending action member comprising:
a movable means having a plurality of articulation portions at its front end side portion and constructed to be bendable;
and a drive power transmitting means constructed to be capable of transmitting the drive power to the movable means, wherein
the first articulation portion on the front end side and the second articulation portion on the rear end side that are constructed to be bendable to the same side and adjoin each other of the plurality of the articulation portions are so constructed that the bending action at the second articulation portion starts after the bending action at the first articulation portion ends, and
the magnitude of a moment required for starting the bending action of the second articulation portion is larger than a moment required for the bending action of the first articulation portion.

13. A manipulator comprising:
a movable means having a plurality of articulation portions at its front end portion and constructed to be bendable;
a drive power generating means constructed to be capable of generating a drive power for operating the movable means; and
a drive power transmitting means constructed to be capable of transmitting the drive power generated by the drive power generating means to the movable means,
wherein
the first articulation portion on the front end side and the second articulation portion on the rear end side that are constructed to be bendable to the same side and adjoin each other of the plurality of the articulation portions are so constructed that the bending action at the second articulation portion starts after the bending action at the first articulation portion ends, and
the magnitude of a moment required for starting the bending action of the second articulation portion is larger than a moment required for the bending action of the first articulation portion.

14. The manipulator according to claim 13, wherein
a pair of forceps members constructed that at least one of them is rotatable is provided at the front end of the movable means and
the pair of the forceps members is capable of gripping a solid object by transmitting a drive power generated by the drive power generating means with the drive power transmitting means.

15. A bending action member comprising:
a movable means constructed that its front end side portion is bendable; and
a drive power transmitting means constructed of at least one link member capable of transmitting a drive power to the movable means, wherein
a first joint portion is constructed of an end on an opposite side to the front end side of the link member, and
the first joint portion is constructed to be connectable with a drive power generating means for generating the drive power and the drive power transmitting means is so constructed to be capable of transmitting the drive power from the drive power generating means to the movable means.

16. The bending action member according to claim 15, wherein
the movable means has a plurality of articulations, and
the first articulation portion on the front end side and the second articulation portion on the rear end side that are constructed to be bendable to the same side and adjoin each other of the plurality of the articulation portions are so constructed that the bending action at the second articulation portion starts after the bending action at the first articulation portion ends.

17. The bending action member according to claim 15 or 16, wherein
the first joint portion has a projecting portion, and
the projecting portion is capable of being fitted to a fitting hole made in a second joint portion composed of elastic body that has the fitting hole and urges substantially perpendicularly to the transmitting direction of the drive power.

18. An actuator constructed to be capable of engaging/disengaging a bending action member provided with a movable means at its front end side portion and having a first joint portion, and comprising a drive power generating means capable of generating a drive power for operating the movable means, wherein
the drive power generating means having a second joint portion containing an elastic body for urging substantially perpendicularly to the transmitting direction of the drive power and having a fitting hole,
the second joint portion is connectable with the first joint portion by advancing substantially linearly along the direction of the drive power and fitting the fitting hole to a projecting portion while applying an urging force by the elastic body to the first joint portion having the projecting portion capable of being fitted to the fitting hole.

19. A manipulator comprising:
a bending action member including a movable means constructed that its front end side portion is bendable and a drive power transmitting means capable of transmitting a drive power applied from outside to the movable means; and
an actuator having a drive power generating means capable of generating a drive power for operating the movable means, wherein
the bending action member and the actuator are constructed to be capable of engaging/disengaging each other; the first joint portion provided on the drive power transmitting means and the second joint portion provided on the drive power generating means are provided to be connectable and separable;
the coupling between the first joint portion and the second joint portion is executed after the bending action member and the actuator are jointed together; and
the first joint portion and the second joint portion are separated accompanied by the separating action of the bending action member and the actuator.

20. The manipulator according to claim 19, wherein
the second joint portion has an elastic body containing a fitting hole and for urging substantially perpendicularly to the transmitting direction of the drive power;
the first joint portion includes a projecting portion capable of being fitted to the fitting hole; and
the second joint portion is advanced substantially linearly along the transmitting direction of the drive power by the drive power generating means and fitted to the fitting hole while generating the urging force to the projecting portion, so that the first joint portion and the second joint portion are coupled to connect the drive power transmitting means with the drive power generating means.
